(19) **Europäisches Patentamt**

**European Patent Office**

**Office européen des brevets**

(11) **EP 1 645 276 A1**

(12) **EUROPEAN PATENT APPLICATION**

(43) Date of publication:
**12.04.2006 Bulletin 2006/15**

(51) Int Cl.:
*A61K 31/519* (2006.01)   *A61K 31/52* (2006.01)
*A61K 31/198* (2006.01)   *A61P 27/16* (2006.01)
*A61P 25/28* (2006.01)

(21) Application number: **04077784.9**

(22) Date of filing: **08.10.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **Wageningen Centre for Food Sciences 6703 GW Wageningen (NL)**

(72) Inventors:
• **Durga, Jane**
  **6706 BL Wageningen (NL)**

• **Verhoef, Petra**
  **2661 PX Bergschenhoek (NL)**

(74) Representative: **van Westenbrugge, Andries et al Nederlandsch Octrooibureau,**
**J.W. Frisolaan 13,**
**P.O. Box 29720**
**2502 LS  Den Haag (NL)**

(54) **Treatment of neurodegenerative disorders**

(57)    The present invention is dedicated to the treatment of neurodegenerative disorders, in particular age related cognitive decline and age related hearing loss. These disorders can be alleviated by increasing 10-formyltetrahydrofolate or increasing thymidylate synthesis. The invention is of particular benefit to subjects that are MTHFR 677CT heterozygote and MTHFR 677CC homozygote.

The substance that increases 10-formyltetrahydrofolate or increases thymidylate synthesis is selected from 10-formyltetrahydrofolate, 5-formyltetrahydrofolate, 5,10-methenyltetrahydrofolate, 5,10-methylentetrahydrofolate, methionine, S-adenosylmethionine and poly-glutamate forms of 10-formyltetrahydrofolate, 5-formyltetrahydrofolate, 5,10-methenyltetrahydrofolate and 5,10-methylentetrahydrofolate and the substance may be in an acid or salt or ester form.

EP 1 645 276 A1

## Description

FIELD OF THE INVENTION

The present invention is in the field of neurodegenerative disorders, like age-related cognitive decline and age related hearing loss.

BACKGROUND OF THE INVENTION

Folate is essential for many physiological functions. Low concentrations of folate have been linked to a multitude of diseases and chronic conditions, like vascular disease, dementia and age-related hearing loss (Lucock, *BMJ* 2004;328:211-214; Blom, *Homocysteine in health and disease.* Cambridge, Cambridge University Press, 2001, pp 331-340). The traditional function of folate is that it accepts and donates one-carbon units (see the figure).

Folate is present almost ubiquitously in natural foods. Dietary patterns are known to change with age, and aging has been associated with lower folate intake and low folate concentrations. In addition to poor dietary habits, aging may influence one's folate metabolism. With increasing age, the activity of enzymes involved in folate metabolism may decrease and the incidence of malabsorptive intestinal disease, like atrophic gastritis increases. Furthermore, folate metabolism is impaired as a consequence of secondary nutrient deficiencies in iron and vitamins $B_6$ and $B_{12}$. The bulk of research on folate is attributed to its role with homocysteine, an amino acid formed in cells when the essential amino acid, methionine, is catabolized (Lucock, *vide supra).* Folic acid supplementation and extra dietary folate intake from the food may decrease concentrations of plasma homocysteine.

Aging is associated with increased risk of neurodegenerative disorders and an increase in their risk factors, such as decreased concentrations of folate and increased concentrations of homocysteine. Observational epidemiology has shown an association of low folate and high homocysteine concentrations with poor cognitive performance (Calvaresi and Bryan, *J.Gerontol.B.Psychol.Sci.Soc.Sci.* 2001; 56:327-339; Selhub et al. *Am.J.Clin.Nutr.* 2000;71:614S-620S). However, evidence from trials on the effect of folic acid supplementation on cognitive function in the general population is still inconclusive (Malouf et al., *Cochrane.Database.Syst.Rev* 2003;CD004514). Many trials employed small study populations, lacked a control group or placebo treatment, allocated multiple interventions such that the effect of folate can not be delineated or employed rather insensitive tests, like the Mini-Mental Screening Examination (Rapin et al., *Trends Biomed.Gerontol.* 1988;1:221-223; Heseker et al. *Bibl.Nutr.Dieta.* 1995; 43-55; Fioravanti et al, *Archives Of Gerontology And Geriatrics* 1997;26:1-13; Nilsson et al. *Int.J. Geriatr. Psychiatry* 2001;16:609-614; Bryan et al. *J.Nutr.* 2002;132:

1345-1356; Toole et al. *JAMA* 2004;291:565-575; Clarke et al. *J Intern Med* 2003;254:67-75).

Age-related hearing loss is a bilateral sensorineural hearing loss initially affecting the higher frequencies and subsequently extending towards the lower frequencies. Decline in hearing acuity may begin in the third and fourth decade of life. Age accounts for 10% of the variation in age-related hearing loss, thus factors that vary with age may play an etiological role in age-related hearing loss. Age-related changes in vascular and neural tissue of the cochlea are thought to contribute to age-related hearing loss (Seidman et al. *Ageing Res.Rev.* 2002; 1:331-343; Cortopassi and Hutchin, *Hear.Res* 1994;78: 27-30).

Apart from atherogenic properties, homocysteine is considered to have neurotoxic properties, as well, which may adversely influence the highly innervated cochlea. For example, homocysteine may act as an agonist at the glutamate site of the N-methyl-D-aspartate receptor (Lipton et al. *Proc.Natl.Acad.Sci. U.S.A.* 1997; 94:5923-5928). Poor hearing has been associated with over excitation of N-methyl-D-aspartate receptors (Puel et al. *Hear.Res.* 1991;51:255-264). However, recently homocysteine was only marginally associated with hearing levels in 91 audiological patients and in the same study no association of folate and vitamin $B_{12}$ with hearing levels was reported (Berner et al. *Acta Oto-Laryngol.* 2000;120:633-637). In contrast to the very weak association found between homocysteine and hearing levels, determinants of elevated concentrations of homocysteine, such as low concentrations of folate and vitamin $B_{12}$ have been inversely associated with hearing levels in women with age-related hearing loss (Berner, vide supra; Houston et al. *Am.J.Clin.Nutr.* 1999;69:564-571). Although age-related hearing loss is one of the most prevalent chronic conditions affecting the elderly, there has been little research on the link with nutrition. No trials have been conducted to determine whether an increase in folate concentrations is associated with improvement in hearing thresholds.

Approximately 10% of the Dutch population has a cytosine-to-thymidine transition in the methylenetetrahydrofolate reductase (MTHFR) gene at nucleotide 677. This mutation results in an alanine-to-valine substitution in the MTHFR protein and leads to a decrease in MTHFR activity compared with the wild type enzyme (Frosst et al. *Nat.Genet.* 1995;10:111-113). MTHFR 677TT homozygotes compared with CC homozygotes are thought to have a higher formyl : methyl tetrahydrofolate derivative ratio, (Bagley and Selhub, *Proc.Natl.Acad.Sci. U.S.A.* 1998;95:13217-13220) hence a lower methylation capacity as indicated by elevated concentrations of homocysteine, especially pronounced when folate concentrations are low (Jacques et al. *Circulation* 1996;93:7-9). The common 5,10-methylenetetrahydrofolate reductase 677C→T polymorphism may give insight into the relation between folate and cognitive performance. Few studies have reported on the nature of the association of MTHFR C677T polymorphism with cognitive function. These studies were small and did not detect an association of MTHFR C677T polymorphism with high intelligence in children or in older adults (Barbaux et al. *Neuroreport* 2000;11:1133-1136; Visscher et

Underlying the present invention was the assessment of the association of folate and homocysteine with age-related disease. From the prior art in the field it follows that the association between folate and cognitive performance may be explained by increased concentrations of plasma homocysteine, a possible neurotoxic substance (Riggs et al. *Am.J.Clin.Nutr.* 1996;63:306-314; McCaddon et al. *Dement.Geriatr.Cogn.Disord.* 2001;12: 309-313; Teunissen et al. *Neurobiol.Aging* 2003;24: 893-902; Seshadri et al. *N.Engl.J.Med.* 2002;346: 476-483). Both low folate concentrations and elevated homocysteine concentrations have been associated with increased risk of vascular disease, (Voutilainen et al, *Circulation* 2001;103:2674-2680; the Homocysteine Studies Collaboration: Homocysteine and risk of ischemic heart disease and stroke - A meta-analysis. *JAMA* 2002; 288:2015-2022) which in turn has been implicated in decreased cognitive performance. Further it can be hypothesized that an elevated concentration of homocysteine and its determinants, like the MTHFR 677TT genotype and decreased concentration of folate and, are associated with poor hearing

The association of folate status with cognitive performance on a battery of tests probing different neuropsychological domains that tend to decline with age and hearing thresholds in middle-aged and elderly men and post-menopausal was studied.

Also, the relationship between MTHFR C677T polymorphism and cognitive performance and the relationship between MTHFR 677TT genotype and hearing were examined.

SUMMARY OF THE INVENTION

Surprisingly it was found that subjects with the MTHFR 677TT genotype had better cognitive performance and hearing levels than subjects with the CC or CT genotype. The markers on which this finding is based are explained in more detail in the Examples, together with possible explanations for this finding. The implication of this finding, and that is also the subject of this invention, is that age-related cognitive decline and age-related hearing loss, or more in general neurodegenerative disorders, can be treated or prevented by mimicking in a subject the phenotype of the MTHFR 677TT genotype.

One characteristic phenotype of the MTHFR 677TT genotype is the presence of a greater formyl folate to methyl folate ratio compared to MTHFR 677CT or MTHFR 677CC subjects. In particular, the phenotype of the MTHFR 677TT genotype shows increased levels of 10-formyltetrahydrofolate, as can be measured in erythrocytes, and/or increased thymidylate synthesis.

Thus the invention concerns a method for the treatment or prevention of neurodegenerative disorders in a subject, said method comprising administering to said subject an effective amount of a substance that increases 10-formyltetrahydrofolate or increases thymidylate synthesis. In other words the invention concerns the use of a substance that increases 10-formyltetrahydrofolate or increases thymidylate synthesis for the preparation of a composition for the treatment or prevention of a neurodegenerative disorder in a subject.

DETAILED DESCRIPTION OF THE INVENTION

MTHFR stands for methylenetetrahydrofolate reductase, or more precise 5,10-methylenetetrahydrofolate reductase.

Hereinbelow the wording 'a substance that increase 10-formyltetrahydrofolate or increases thymidylate synthesis' is also abbreviated to 'a substance'.

The term "folate" is used for convenience, but is intended to also encompass the folic acid.

Throughout this description and in the appending claims the word "comprising" and variations of the word such as "comprise" and "comprises" is to be interpreted as specifying the presence of the stated parts, steps, additives or components, but does not exclude the presence of one or more additional parts, steps, additives or components. A composition comprising a substance may thus comprise additional active or functional components.

The present invention concerns a method for the prevention or treatment of neurodegenerative disorders. In the context of this invention the term "neurodegenerative disorders" is intended to encompass a variety of disorders that may benefit from the present method for treatment or prevention including diabetes, insulin resistance, schizophrenia, MELAS (stands for mitochondrial myopathy, encephalopathy, lactic acidosis and stroke-like episodes), renal disease, Parkinson disease and other types of dementia (like Frontal Temporal Dementia, Alzheimer's disease, vascular dementia, mild cognitive impairment, Kearns-Sayre syndrome, multiple sclerosis, prevention of neural tube defects and down syndrome, Parkinson's disease, aggressiveness and anxiety, depression, age-related hearing loss and cognitive decline, in particular age-related cognitive decline. Preferably the invention concerns the prevention or treatment of age-related hearing loss or age-related cognitive decline.

The cognitive functions with the greatest variability with age are memory and speed-related functions. Thus in one embodiment the invention concerns the prevention or treatment of cognitive decline which is related to memory and speed-related functions.

Substances that increase 10-formyltetrahydrofolate or increases thymidylate synthesis are for instance glycine, 10-formyltetrahydrofolate, 5-formyltetrahydrofolate, 5,10-methenyltetrahydrofolate, 5,10-methylenetetrahydrofolate, methionine and S-adenosylmethionine. In one embodiment the substance that is administered or that is used for the preparation of a composition according to the invention is selected from glycine, 10-formyltetrahydrofolate, 5-formyltetrahydrofolate, 5, 10-methenyltetrahydrofolate, 5,10-methyleentetrahydrofolate, methionine and S-adenosylmethionine. The folate derivatives 10-formyltetrahydrofolate, 5-formyltetrahydrofolate, 5,10-methenyltetrahydrofolate, 5,10-methylenetetrahydrofolate may also be used or administered in their poly-glutamate form, and the term folate includes folic acid.

Also the term "folate" indicates that the molecule at stake may be in its salt or ester form. The ester form may be readily hydrolysed *in vivo* and such esters are known to the skilled person. Common salts of folate derivatives are for instance the calcium salt, magnesium salt or disodium salt.

Preferably, in the method of the invention a daily amount of between 0.01 and 500 mg substance per kg metabolic weight of the subject is administered. The effective dose will vary on the activity of a substance and its bioavailability. For instance for the folate derivatives it is known that poly-glutamates are less bioavailable than mono-glutamates. The metabolic weight of a subject is calculated with the following formula:

$$\text{metabolic weight} = (\text{body weight})^{0.75}$$

For a human, the daily amount of folate derivative administered is for instance between 0.01 and 100 mg per kg body weight.

In a further embodiment, the present method comprises the administration of a dosage comprising between 1 mg and 6 g substance. The effective dose may be subdivided into several smaller dosages and administered for example in two, three or more portions per day.

The present invention, being particularly aimed at the treatment, or prevention of neurodegenerative disorders, may be manifested in the form of products, such as nutritional supplements or functional foods, advertised to treat or prevent undesirable conditions of aging and/or the treatment or prevention of symptoms thereof, such as hearing loss and cognitive decline. Nutritional supplements labelled with terminology pointing to the above treatment are encompassed by the present method. Such terminology includes for example "hearing loss", "general slowness," "loss of memory", "forgetfulness", "slowed reaction time," "increase cognitive performance", "increase cognitive functions".

Preferably, the present invention comprises the administration of a substance incorporated in a pharmaceutical preparation or nutritional supplement. This preparation preferably comprises or consists of the substance optionally in the form of a nutritionally and pharmaceutically acceptable salt or ester and a pharmaceutically and/or nutritional acceptable carrier.

Packaged nutritional supplements and dietary products, which have been provided with labels that explicitly or implicitly direct the consumer towards the use of said supplement or product in accordance with one or more of the uses or methods described herein, are encompassed by the present invention.

In accordance with the present method the substance is preferably administered enterally, more preferably orally. The substance used in the present method can be applied in any suitable form, such as meals, bars, pills (e.g. capsules, tablets or caplets), gels, biscuits and drinks. However, the substance may also be in a form for other methods of administration such as a fluid for injection.

According to a further preferred embodiment the substance is provided in a unit dosage form. The term unit dosage form refers to a physically discrete unit suitable for unitary administration to a subject, wherein each unit contains a predetermined quantity of substance and a pharmaceutically acceptable carrier.

The aforementioned unit dosage form is preferably in a solid or semisolid form, more preferably in the form of an oral dosage unit, which term includes capsules, tablets, microparticles and microspheres.

The folate derivatives used in the present invention may be in a for stability unfavourable oxidation state. Therefore in one embodiment a stabiliser is present that maintains the folate derivative in its oxidation state prior to release of the active ingredient at the site of action in a subject. It may be beneficial to use the folate derivatives in microcapsules together with a stabiliser to prepare the composition according of the invention or in a composition for administration.

The folate derivative used in the present invention may be modified in environments of the upper intestinal tract and is therefore preferably orally administered in a unit dosage form that is coated with a substance that can withstand the enteric environment (an enteric coating) to prevent the decrease of its activity.

In view of its commercial availability the use of 5-formyltetrahydrofolate, in a salt or ester form, particular the calcium salt, or 5-formyltetrahydrofolic acid (folinic acid) is preferred.

In a further aspect, the present invention provides a container that comprises multiple active substance containing dosages. The container bears a label, which indicates that the dosages should be ingested by an elderly person for the benefit of hearing and or cognition or terms indicative of such functions. The container preferably contains between 2 and 250 dosages, more preferably between 7 and 150 dosages, even more preferably between 25 and 100 dosages per container.

Apart from a substance that increases 10-formyltetrahydrofolate or increases thymidylate synthesis in a suitable dosage, a nutrition composition preferably comprises carbohydrates and/or proteins and/or lipids suitable for human consumption and preferably taking into account the nutritional requirements of elderly subjects. Thus, the compositions may comprise additional components, such as proteins, carbohydrates, vitamins, minerals, trace elements, amino acids, other biologically or pharmaceutically active compounds, carriers, stabilisers, flavourings, probiotics, prebiotics, and the like. Characteristic for the nutritional requirements of the elderly is a relatively high fiber content and a relatively modest energy content as provided by protein, carbohydrate and fat. Further a sufficient amount of vitamin D is preferred.

Target groups that are of particular interest for the present invention are subjects that are heterozygote MTHFR 677CT or MTHFR 677CC and/or subjects that are 50 years of age or more.

Also food products that are fortified with a substance that increases 10-formyltetrahydrofolate or increases thymidylate synthesis for the purpose according to the invention are intended to fall under the scope of this invention.

DESCRIPTION OF THE FIGURE

The figure shows metabolism and function of folate Abbreviations. DNA—CpG—DNA DNA CpG island; THF tetrahydrofolate; DHF dihydrofolate; MS methionine synthase; CβS cystathionine β-synthase; DHFR dihydrofolate reductase; SHMT serine hydroxymethyltransferase; MTHFR methylenetetrahydrofolate reductase; TS thymidylate synthase; dUMP deoxyuridylate; dTMP thymidylate; $C_{ox}$ oxidized cytochrome c; $C_{red}$ reduced cytochrome c.

EXAMPLES

Introduction

Many individuals experience neurodegenerative disorders as they grow older. In order to slow down or prevent this kind of disorders, in particular age related cognitive decline and age related hearing loss, one needs to understand the factors that explain differences with age between people. Nutritional factors that decrease with increasing age, like decreasing B vitamin concentrations, may help explain variations in the (rate of) occurrence of neurodegenerative disorders. The B vitamins, specifically folate, have been implicated in neurological disorders. Consumption of a folate-deplete diet for three months was associated with forgetfulness, paranoia and irritability (Herbert *Transactions of the Association of American Physicians* 1962;75:307-320). These symptoms resided once folate in the diet was replenished. Likewise, in patients with deficient B vitamin levels or inborn errors of one-carbon metabolism, folate supplementation improved cognitive function (Botez et al. *Psychol.Med.* 1984;14:431-437; Pasquier et al. *J.Neurol.Neurosurg.Psychiatry* 1994;57:765-766; Reynolds, *Lancet* 1967;1:1086-1088; Botez et al. *Eur.Neu-*

*rol.* 1977;16:230-246; Enk et al. *Scand.J.Haematol.* 1980;25:63-66; Freeman et al. *N.Engl.J.Med.* 1975;292: 491-496; Manzoor and Runcie *Br.Med.J.* 1976;1: 1176-1178; Strachan and Henderson *Q.JMed.* 1967;36: 189-204). No trials have been conducted to determine whether an increase in folate concentrations is associated with improvement in hearing thresholds.

Here the relationship between low folate and high homocysteine concentrations, on the one hand, and cognitive performance and hearing, on the other hand, are presented. The data used are obtained from the FACIT trial, which is an acronym for Folic Acid and Carotid Intima-media Thickness. The FACIT trial investigates the effects of 0.8 mg folic acid supplementation for three years on carotid intima-media thickness, cognitive function and hearing.

Methods - Cognition

*Subjects*

Data come from 819 men and post-menopausal women aged 50 to 70 years participating in the Folic Acid and Carotid Intima-media Thickness (FACIT) study, a single-center double blind randomized control trial investigating whether folic acid supplementation reduces the risk of cardiovascular disease, as measured by carotid intima-media thickness. Major exclusion criteria were homocysteine <13 $\mu$mol/L, vitamin $B_{12}$ <200 pmol/L, renal or thyroid diseases and use of B vitamin supplements or medications that influence folate metabolism or atherosclerotic progression (e.g. lipid-lowering and hormone replacement therapies).

*Cognitive function*

Cognitive function was assessed using five separate tests: the Word Learning test, the Concept Shifting test, the Stroop Color-Word test, Verbal Fluency test and the Letter Digit Substitution test. The *Word Learning task* measures the storage and retrieval of newly acquired verbal material. Fifteen monosyllabic words are visually presented in a sequence, for two seconds each. The subjects are asked to recall the words immediately after each of the 3 presentation trials (immediate recall). Twenty minutes after the last trial, subjects are again prompted to recall the words (delayed recall). The number of correctly repeated words per trial is recorded. The *Concept Shifting task* estimates the ease of switching between two psychological concepts, as an index of perceptual planning and evaluation. First subjects are asked to cross out empty circles, then circles with numbers in chronological order and fmally circles with letters in alphabetical order, randomly arranged in a circle. The final task is to alternately cross out circles with either numbers or letters, in chronological and alphabetical order. The time needed to complete the tasks is recorded. The *Stroop Color-Word test* measures selective attention and susceptibility to behavioral interference. Subjects are initially asked to read the name of colors (red, blue, green, yellow) and then name color blocks. Finally, subjects are asked to name the color of the ink rather than the word. The time needed to complete the tasks is recorded. The *Verbal Fluency test* measures the ability to draw on one's encyclopedic memory in a strategy-based manner. The subject is asked to name as many animals as possible in one minute. The number of different animals named is recorded. The *Letter Digit Substitution test* assesses general speed of visual information processing. Nine different letters are coupled with nine different numbers in a key on the top of the form. The subject is asked to copy the corresponding letter by each number as quickly as they can. The number of correctly filled in letters in ninety seconds is recorded. These tests were derived from the assessment protocol of the Maastricht Aging Study, a prospective study of the determinants of cognitive aging; for more extensive descriptions of these tests see Jolles et al. (AnonymousMethods II: Measurements, in Jolles J, Houx PJ, van Boxtel MPJ, Ponds RWHM (eds): *Maastricht Aging Study: Determinants of cognitive aging* . Maastricht, Neuropsych Publishers, 1995, pp 25-38).

We included the Mini-Mental State Examination (Folstein et al. *J.Psychiatr.Res.* 1975;12:189-198), a widely used dementia-screening tool, to allow for comparison of our study population with other study populations and to exclude unreliable tests scores from subjects with suspected severe cognitive dysfunction (defined as <24 out of 30 points). All participants underwent the measurements after an overnight fast broken by a small snack consisting of a glass of juice and breakfast muffin. Trained research assistants administered the test during a 40-minute session to all but one participant; he was excluded from the data analyses.

*Blood measurements*

Fasting venous blood was collected, directly processed and stored at -80°C. Folate and vitamin $B_{12}$ were measured using a chemiluminescent immunoassay (Immulite® 2000, Diagnostic Products Corporation). Erythrocyte folate was determined in duplicate and the average was taken to reduce measurement error. Serum creatinine and lipids were determined using Hitachi® 747 (Roche). We defined hypercholesterolemia as total cholesterol >6.5 mmol/L, high-density lipoprotein cholesterol (HDL) <0.9 mmol/L or use of lipid-lowering medication. Homocysteine was determined with HPLC and fluorimetric detection (Ubbink et al. *J.Chromatogr.* 1991;565: 441-446). Vitamin $B_6$ was measured by HPLC. The C677T MTHFR genotype was determined by polymerase chain reaction of DNA and restriction enzyme digestion with *Hin*F1. Intra-assay and inter-assay variation coefficients for laboratory analyses were <15%.

*Carotid ultrasound*

Carotid intima-media thickness, a valid surrogate marker of vascular disease and a structural marker of atherosclerosis, was measured using high-resolution B-mode ultrasonography with a 7.5 MHz linear-array transducer (ATL UltraMark IX). Longitudinal images of the distal common carotid arteries were obtained at four predefined angles of 30° steps (90° to 180° on the right side and 270° to 180° on the left side). Images were frozen on the top of the R-wave of the electrocardiography and recorded on VHS tape. The maximum distance of the near and far wall of the distal 10 mm of the right and left common carotid artery was determined using an automated edge detection program. Carotid distension, a marker of arterial stiffness believed to increase as a result of aging and atherosclerosis, was measured using M-mode sonography. An M-line was placed at an angle deemed by the sonographer to give the best visualization of the arterial walls. The sonographer recorded carotid artery pulsation for approximately three seconds. The relative distension of the carotid artery was calculated as the absolute change in lumen diameter relative to its end-diastolic diameter was calculated. The average of three measurements per side was calculated. Ultrasound examinations were performed in duplicate within three weeks; the average was used for analyses.

*Other measurements*

A self-reported medical history, including current drug use and family history of premature vascular disease (onset <60 years in first degree family) and smoking were attained by questionnaire and reviewed by a research assistant. A participant was considered to have prevalent vascular disease if he had been clinically diagnosed with angina pectoris, myocardial infarction, arrhythmia, stroke, or peripheral arterial disease or if he had undergone certain palliative procedures (balloon angioplasty, coronary bypass surgery or aortic aneurysm surgery). Education was classified according the Dutch Central Bureau of Statistics and grouped according to highest attained level: primary education, junior vocational training or senior vocational/academic training. Height and weight were measured and body mass index ($kg/m^2$) was calculated. Blood pressure was measured using an automated meter (Dinamap® Compact Pro 100, General Electric) in supine position, while the participant underwent the ultrasound examination. The average of eight measurements was taken. We defined hypertension as systolic blood pressure ≥160 mmHg, diastolic blood pressure ≥95 mmHg or use of anti-hypertensive medication. Mean arterial pressure was calculated by the following formula: diastolic blood pressure + 1/3 (systolic blood pressure - diastolic blood pressure). A food frequency questionnaire estimated folate intake and alcohol intake in the past three months. Two typists, blinded to study information, separately entered all data.

**Statistical analysis**

The domains—sensorimotor speed, complex speed and memory—were constructed by combining the results of Concept Shifting test, Stroop Color-Word test and Word learning test. In order to combine sub-tests, Z-scores were calculated. Sensorimotor speed index is a measure for basic speed and is compromised of the first two trials in the Concept Shifting test and Stroop Color-Word test, whereas complex speed index, a measure of cognitive flexibility and the ability to switch between different tasks, is composed of the final trials of the same tests. The verbal memory index is calculated using the total and the maximum of the three immediate recall trials and the delayed recall score. See van Boxtel et al for an extensive description of this data reduction method (van Boxtel et al. *J.Gerontol.A.Biol.Sci.Med.Sci.* 1998;53: M147-M154). The Letter Digit Substitution test and Verbal Fluency test were not included in compound measures, however, for ease of comparison we converted the raw scores to Z-scores as well.

For each participant, we averaged the homocysteine concentrations measured at screening and at baseline and used this average in the analyses. To investigate the association between folate and cognitive performance, the mean adjusted cognitive performance was calculated based on folate status: deficiency (erythrocyte folate ≤305 nmol/L; serum folate ≤7 nmol/L), low-normal (erythrocyte folate 306-500 nmol/L; serum folate 8-11 nmol/L) and high-normal (erythrocyte folate ≥501 nmol/L; serum folate ≥12 nmol/L). Adjustment was done initially for age, sex and education level and then for other possible confounders. Possible confounders were hypertension, total cholesterol, HDL, hypercholesterolemia, serum creatinine, apoE ε4 allele, diabetes mellitus, body mass index, current smoking, smoking pack years and alcohol intake. To examine whether the association between folate and cognitive performance may be mediated by homocysteine or vascular disease risk, we included homocysteine or carotid intima-media thickness or carotid distension. We included mean arterial pressure as a covariate in the analyses involving distension. Because the MTHFR 677T allele is associated with hyperhomocysteinemia mainly when folate concentrations are low (de Bree A, et al. *Am.J.Clin.Nutr.* 2003;77: 687-693), we stratified the population based on folate status and studied the relationship between genotype and cognitive performance levels within these strata. The latter analyses were adjusted for age, sex and education level using analysis of covariance. Statistical significance was defined as $p<0.05$ (two-tailed). Statistical analyses were performed using SPSS 11.0 for Windows.

**Results**

Nine percent of the participants had deficient levels of serum folate, erythrocyte folate and vitamin $B_6$ according to the American Institute of Medicine. The MTHFR 677T allelic distribution did not significantly differ from the calculated expected distribution, assuming a Hardy-Weinberg equilibrium. Seven subjects scored <24 points out of 30 on the Mini-mental state examination, which may indicate a cognitive disorder. The associations of folate status and MTHFR 677TT genotype with cognitive performance were similar regardless if these subjects were excluded from the analyses, thus we presented the results from the total population. Second to educational level, age was a strong determinant of performance on cognitive tests. The association with age was greater for complex speed and information processing and weakest for Verbal Fluency test, after adjustment for conventional risk factors of vascular disease (sensorimotor speed β=-0.038 (standard error 0.005), $p<0.001$; complex speed β=-0.040 (0.005), $p<0.001$; memory β=-0.029 (0.006), $p<0.001$; Letter Digit Substitution test β=-0.043 (0.006), $p<0.001$; Verbal Fluency β=-0.018 (0.006), $p=0.005$).

*Folate and cognitive performance*

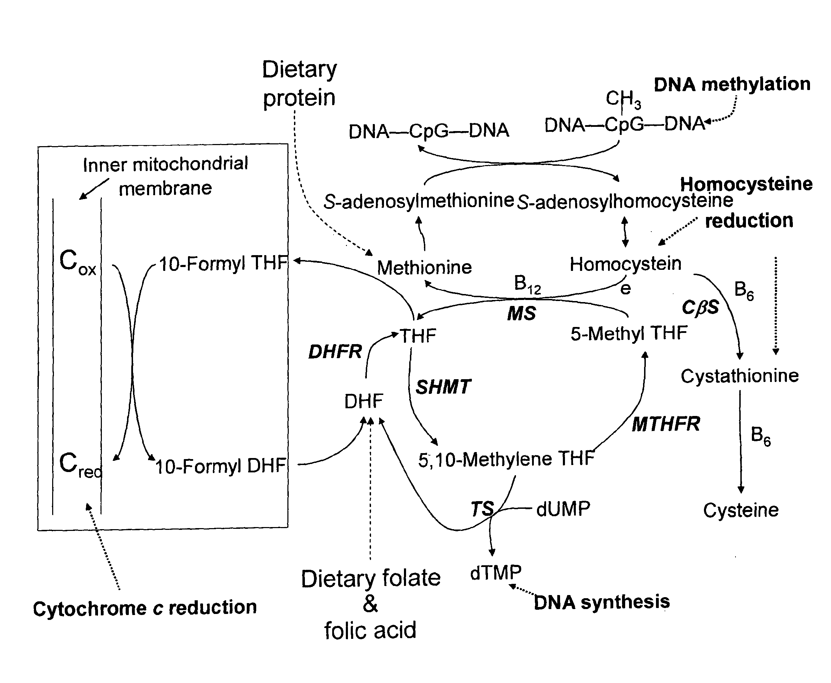

Erythrocyte folate deficiency was associated with poor performance on a variety of cognitive tests despite the small number of individuals with deficient erythrocyte folate concentrations. In crude analyses, subjects with deficient concentrations of erythrocyte folate performed poorer on all cognitive tests than subjects with high-normal concentrations, however, this did not reach statistical significance for the Verbal Fluency test. After adjustment for age, sex and educational level, the associations remained for sensorimotor speed, complex speed and memory (mean difference in Z-scores between deficient vs. high-normal concentrations: sensorimotor speed -0.33, 95% CI -0.65 to -0.01; complex speed -0.52, 95% CI -0.88 to -0.16; memory -0.52, - 0.92 to -0.11). The association of erythrocyte folate with cognitive performance remained statistically significant for complex speed and memory after adjustment for conventional risk factors of vascular disease (mean difference in Z-scores between deficient vs. high-normal concentrations: sensorimotor speed -0.27, 95% CI -0.60 to 0.07; complex speed -0.45, 95% CI -0.82 to -0.08; memory -0.50, 95% CI -0.92 to - 0.10) (Figure 4.1).

We examined whether the association between deficient concentrations of erythrocyte folate and cognitive performance might be mediated by elevated concentrations of homocysteine or increased risk of vascular disease, the latter estimated by increased intima-media thickness or low distension. Age and sex adjusted erythrocyte folate concentrations were correlated with homocysteine concentrations (r=-0.26, $p<0.001$) and with intima-media thickness (r=-0.08, $p=0.019$), but not with distension. In addition, homocysteine concentrations were correlated with sensorimotor and complex speed (partial correlation coefficient: sensorimotor speed r=-0.07, $p=0.040$; complex speed r= -0.07, $p=0.049$) and intima-media thickness was correlated with sensorimotor and complex speed and the Letter Digit Substitution test (partial correlation coefficient: sensorimotor speed r=-0.09, *p*=0.017; complex speed r=-0.09, *p*=0.014; Letter Digit Substitution test r=-0.06, *p*=0.083) independent of conventional risk factors for vascular disease. Addition of variables on vascular disease prevalence or familial history of premature vascular disease did not significantly affect the association of intima-media thickness and cognitive performance. The association between erythrocyte folate and complex speed was independent of homocysteine concentrations (mean difference deficient vs. high-normal concentrations: complex speed -0.39, 95% CI -0.76 to -0.01) and independent of intima-media thickness or other markers of vascular disease (i.e., vascular disease prevalence and familial history of premature vascular disease) (mean difference in Z-scores between deficient vs. high-normal concentrations: complex speed -0.44, 95% CI -0.80 to -0.07).

*MTHFR and cognitive function*

Despite similar folate intake, subjects with the MTHFR 677TT genotype had higher homocysteine concentrations and lower serum folate concentrations than CC or CT subjects (homocysteine 15.3 vs. 14.2 $\mu$mol/L, *p*<0.001 ; serum folate 11 vs. 13 nmol/L, *p*=0.001). No other study variables differed between the MTHFR C677T genotypes. MTHFR 677TT homozygotes tended to have higher cognitive performance scores on all tests except Verbal Fluency than CC homozygotes, with CT heterozygotes having intermediate values. In subjects with a folate status below that of the population median, having the MTHFR 677TT genotype was often associated with better performance on sensorimotor and complex speed. Folate intake, in contrast to blood concentrations of folate, was not likely to be influenced by MTHFR C677T genotype.

When the population was stratified on the basis of folate intake, MTHFR 677TT homozygotes in the upper half of the folate intake distribution tended to show better cognitive performance compared with their CC or CT counterparts. Folate intake was weakly correlated with blood folate levels (erythrocyte folate r=0.11, *p*=0.002; serum folate r=0.16, *p*<0.001, adjusted for age, sex and MTHFR C677T genotype)

**Discussion**

We have shown that deficient levels of erythrocyte folate were associated with poor cognitive domains, i.e. complex speed and memory, independent of risk factors for vascular disease, including homocysteine and intima-media thickness. Contrary to our expectations, subjects with the MTHFR 677TT genotype often performed better on cognitive tests. When we examined the relation between MTHFR C677T polymorphism and cognitive performance stratified by folate status, having the MTHFR 677TT genotype was associated with better performance on sensorimotor and complex speed in individuals with a low folate status. Folate intake, unlike serum or erythrocyte folate concentrations, is unlikely to be influenced by the MTHFR C677T genotype and hence is more appropriate to examine effect modification due to folate. However, the positive association of MTHFR 677TT genotype and cognitive performance was strongest among those with high folate intake rather than low folate intake.

In our study deficient levels of erythrocyte folate were linked to poor performance on tests probing cognitive domains susceptible to age-related cognitive decline, like sensori-motor and complex speed and memory. MTHFR C677T genotype is unlikely to affect other factors than folate metabolism. MTHFR 677TT homozygosity was paired with better cognitive performance on all tests, except Verbal Fluency. When folate status is low, phenotypic expression of the MTHFR 677TT genotype is most pronounced, i.e. concentrations of homocysteine increase.

A possible protective effect of the MTHFR 677TT genotype may lie in its greater relative one-carbon commitment to *de novo* synthesis of nucleotides and an increase in formyl-folate derivatives relative to methyl-folate derivatives. In theory, subjects with a greater capacity of thymidylate synthesis and 10-formyltetrahydrofolate generation may have a reduced risk of genomic and mitochondrial DNA damage in two ways. Individuals with the MTHFR 677TT genotype may have increased thymidylate synthesis that may directly reduce DNA mutations by ensuring a smaller pool of uracil. Secondly, MTHFR 677TT homozygotes may have greater amounts of 10-formyltetrahydrofolate that may protect mitochondrial integrity by reducing cytochrome c and may have other antioxidant-like functions in the cytosol and mitochondria.

**Methods - Hearing**

*Subjects*

Data are from 819 men and post-menopausal women aged 50 to 70 years participating in the Folic Acid and Carotid Intima-media Thickness (FACIT) study, a trial investigating whether folic acid supplementation can halt the progression of atherosclerosis. Major exclusion criteria were homocysteine <13 $\mu$mol/L, vitamin $B_{12}$ <200 pmol/L, renal or thyroid diseases and use of B vitamin supplements or medications that influence folate metabolism or atherosclerotic progression (e.g. lipid-lowering and hormone replacement therapies). Ninety subjects were excluded because of hearing loss attributed to middle ear dysfunction (air-bone gap ≥15 dB in either ear) or unilateral hearing loss (a difference in average pure-tone hearing thresholds for 0.5 kHz, 1 kHz, 2 kHz ≥20 dB between the right and left ear).

*Audiometric measurements*

Subjects' ears were examined for excessive cerumen, which was removed if present. Pure-tone air and bone conduction thresholds were obtained with subjects seated in an acoustical booth (Audiofon G, Audiovox Italy) using a portable diagnostic audiometer (Voyager 522). We calculated average pure-tone air conduction thresholds of 0.5, 1 and 2 kHz (PTA-low) and of 4, 6 and 8 kHz (PTA-high). Audiometric testing was done using a variation of the Hughson and Westlake method: thresholds based on ascending responses using up 5 dB, down 10 dB steps (Hughson and Westlake, Manual for program outline for rehabilitation of aural casualities both military and civilian. 1944. Rochester, New York, U.S.A., American Academy of Ophtalmologists and Otologists. (GENERIC) Ref Type: Serial (Book,Monograph)). Contralateral masking was used when necessary. Twenty-nine subjects had a hearing threshold at a number of frequencies that was not measurable by our audiometer (maximum pure-tone air conduction emitted was 100 dB; 2 kHz $n$=1, 4 kHz n=4, 6 kHz $n$=6, 8 kHz $n$=29). These subjects were assigned a hearing threshold of 105 dB, as it was assumed that their hearing threshold was greater than 100 dB.

*Blood measurements*

See above

*Carotid ultrasound*

See above

*Other measurements*

See above

**Statistical analysis**

For each participant, we averaged the homocysteine and vitamin $B_{12}$ concentrations measured at screening and at baseline and used this average in the analyses. The average of the right and left ear PTA hearing threshold was used for the analyses. After transformation (natural log) of the PTA-low and PTA-high distributions to obtain normality, the relation between conventional risk factors of vascular disease and (subclinical) markers of vascular disease and PTA-low and PTA-high were assessed using linear regression. In order to calculate mean hearing thresholds among tertiles of homocysteine and B vitamins, we employed analysis of covariance. Test for trend was extracted from linear regression analyses with the variable of interest entered as a continuous variable. Confounders considered were systolic and diastolic blood pressure, hypertension, diabetes, lipids, hypercholesterolemia, serum creatinine, body mass index, alcohol intake, current smoking status and smoking pack years.

We investigated whether the association of homocysteine with hearing was explained by markers of vascular disease and the association of B vitamins with hearing was explained by homocysteine or markers of vascular disease by subsequently adding these variables into the regression model. We included mean arterial pressure as covariates in the analyses involving distension. Because the MTHFR 677TT genotype is associated with elevated concentrations of homocysteine mainly when folate status is low, we stratified the population by the folate status (population median), and studied the relationship between genotype and hearing levels within these strata. The latter analyses were adjusted for age, sex and education level using ANCOVA. Statistical significance was defined as p <0.05 (two-tailed). Statistical analyses were performed using SPSS 11.0 for Windows.

**Results**

Seventy-two percent of the study participants were male and the average age was $60\pm6$ years. Median homocysteine concentrations were 13.3 μmol/L (range 10.1 to 29.4 μmovL). Nine percent of the participants had deficient levels of serum folate, erythrocyte folate and vitamin $B_6$ according to the American Institute of Medicine. The MTHFR 677T allelic distribution did not significantly differ from the calculated expected distribution, assuming a Hardy-Weinberg equilibrium. Our study population had a higher prevalence of MTHFR 677TT homozygotes than in the general population in The Netherlands (15.7 vs. ~10%, respectively); this is a likely consequence of our homocysteine and vitamin $B_{12}$ inclusion criteria.

*Homocysteine, B vitamins and hearing levels*

The mean age, sex and educational level adjusted hearing threshold of the low and high frequencies per tertile of homocysteine, folate, vitamin $B_{12}$ and vitamin $B_6$ were analysed. Contrary to our hypothesis, the direction of association was such that low concentrations of homocysteine and high concentrations of B vitamins were associated with elevated hearing thresholds for PTA-high and PTA-low. The direct association of serum folate and vitamin $B_{12}$ with PTA-high hearing thresholds was independent of homocysteine concentrations and other risk factors of vascular disease, including prevalent vascular disease and premature vascular disease.

*MTHFR genotype and hearing levels*

The MTHFR 677TT genotype was paired with higher homocysteine concentrations and lower serum folate concentrations, despite similar folate intake. Before adjustment for age, sex and educational level, MTHFR 677TT homozygotes had slightly lower PTA-high hearing thresholds (median [interquartile range] 30 dB [20 to 49 dB]) than subjects with the MTHFR CT and MTHFR CC genotype (CT: 36 dB, 95% CI 23 to 51 dB, TT vs. CT p=0.065; CC: 35 dB 95% CI 23 to 49 dB, TT vs. CC p=0.079). Because the phenotypic expression of the MTHFR C677T polymorphism is most pronounced when folate concentrations are low, we stratified the population based on median folate concentrations. MTHFR 677TT homozygotes with a folate status below that of the population median did not have higher hearing thresholds than CC homo-zygotes. Because folate status is influenced by MTHFR C677T genotype, we also examined the association of MTHFR C677T genotype with hearing levels stratified by folate intake. Similarly, MTHFR 677TT homozygotes with a folate intake below that of the population did not have higher hearing thresholds than CC homozygotes.

It is of interest to note, that subjects in the upper half of the folate status distribution with two MTHFR 677T alleles compared with subjects with either one or no T allele tended to have on average lower hearing thresholds. Furthermore, the association of folate status with hearing thresholds depended on MTHFR C677T genotype: high serum folate concentrations were associated with lower hearing thresholds in MTHFR 677TT homozygotes, whereas high serum folate concentrations were associated with higher hearing thresholds in MTHFR CC homozygotes (PTA-low: CC r=0.12, p=0.033; TT r=-0.12, p=0.187, adjusted for age, sex, educational level). A similar trend was seen in PTA-high.

## Discussion

The primary findings of our study are that an elevated concentration of homocysteine, a novel risk factor for vascular disease, is not associated with hearing. Likewise, determinants of elevated concentrations of homocysteine—low concentrations of B vitamins—were not associated with elevated hearing thresholds. Conversely, high concentrations of serum folate and vitamin $B_{12}$ were associated with poorer hearing in the high frequencies, independent of homocysteine and markers of cardiovascular disease. MTHFR 677TT homozygotes had similar hearing levels as subjects with a C allele when folate status was below the median. However, when folate status was above population median, MTHFR 677TT genotype seemed to offer protection against hearing loss. Low concentrations of serum folate and vitamin $B_{12}$ are independently associated with hearing acuity.

In our population, high serum folate concentrations were associated with better hearing in MTHFR 677TT homozygotes, whereas high serum folate concentrations were associated with poorer hearing in CC homozygotes. Contrary to our a priori hypothesis, subjects with the MTHFR 677TT genotype tended to have better hearing than subjects with the 677C allele. Given the general pattern of our data we can speculate on the pathobiology of sensorineural hearing loss. Our data lend support to the mitochondrial damage theory of age-related hearing loss. This theory purports that damage to the mitochondrial DNA slowly builds up over time and once a critical threshold is attained, the mitochondria are rendered bioenergetically inefficient and cells undergo apoptosis. A possible protective effect of the MTHFR 677TT genotype may lie in its greater relative one-carbon commitment to de novo synthesis of nucleotides and an increase in formyl-folate derivatives relative to methyl-folate derivatives. In theory, subjects with a greater capacity of thymidylate synthesis and 10-formyltetrahydrofolate generation may have a reduced risk of mitochondrial DNA damage in two ways. Individuals with the MTHFR 677TT genotype may have increased thymidylate synthesis that may directly reduce mitochondrial DNA mutations by ensuring a smaller pool of uracil. Secondly, MTHFR 677TT homozygotes may have greater amounts of 10-formyltetrahydrofolate, which may protect mitochondrial integrity by reducing cytochrome c and due to its structural lability may have other antioxidant-like functions.

A shift in the distribution of folate derivatives is not only influenced by polymorphisms in folate-dependent enzymes like MTHFR C677T polymorphism, but also by intracellular folate concentrations. Low folate concentrations are associated with a greater retention of one-carbon moieties for de novo nucleotide synthesis and greater 10-formyltetrahydrofolate concentrations. Thus, in subjects with the MTHFR 677CC or 677CT genotype, lower folate concentrations may be linked to DNA fidelity and increased oxidative phosphorylation of the mitochondria, and may explain why these subjects have better hearing when folate levels are low.

Our original hypothesis was that vascular disease risk factors, elevated homocysteine and decreased folate concentrations were associated with poorer hearing, possibly independent of vascular disease.

In agreement with previous studies, prevalence of vascular disease was weakly associated with poor hearing. In our population neither carotid intima-media thickness nor distension was related to hearing thresholds. Our findings do not support a strong role for vascular disease in the etiology of age-related hearing loss.

## Proof of principle

In a randomized placebo controlled trial a group of elderly subjects between 50 and 70 years of age are given orally folinic acid calcium salt 100 mg/day during 60 days.

At day 0, 1, 7, 14, 30 and 60 cognitive performance and hearing thresholds are determined as described above. None of the subjects shows a decrease in cognitive performance and hearing thresholds. The subjects having cognitive performance and a hearing threshold below average benefit form the folinic acid calcium salt intake by displaying better cognitive performance and a higher hearing threshold.

MTHFR genotype in these subjects is not assessed.

**Claims**

1. Use of a substance that increases 10-formyltetrahydrofolate or increases thymidylate synthesis for the preparation of a composition for the treatment or prevention of a neurodegenerative disorder in a subject.

2. Use according to claim 1 wherein the neurodegenerative disorder is age-related hearing loss.

3. Use according to claim 1 wherein the neurodegenerative disorder is cognitive decline.

4. Use according to claim 3 wherein the cognitive decline is related to memory and speed-related functions

5. Use according to any of claims 1-4 wherein the substance that increases 10-formyltetrahydrofolate or increases thymidylate synthesis is selected from 10-formyltetrahydrofolate, 5-formyltetrahydrofolate, 5,10-methenyltetrahydrofolate, 5,10-methyleentetrahydrofolate, methionine, S-adenosylmethionine and poly-glutamate forms of 10-formyltetrahydrofolate, 5-formyltetrahydrofolate, 5,10-methenyltetrahydrofolate and 5,10-methyleentetrahydrofolate and the substance may be in an acid or salt or ester form.

6. Use according to any of claims 1-5 wherein the substance that increases 10-formyltetrahydrofolate or increases thymidylate synthesis is 5-formyltetrahydrofolic acid or a salt or ester thereof.

7. Use according to any of claims 1-6 wherein the subject is heterozygote MTHFR 677CT or MTHFR 677CC.

8. Use according to any of claims 1-7 wherein the subject is 50 years of age or more.

9. Composition consisting of a substance that increases 10-formyltetrahydrofolate or increases thymidylate synthesis in a subject and a nutritionally and pharmaceutically acceptable carrier and optionally a stabiliser that keeps the substance stable in its oxidation state.

10. Composition according to claim 9 wherein said substance is 5-formyltetrahydrofolic acid or a salt or ester thereof.

**European Patent**
**Office**

## PARTIAL EUROPEAN SEARCH REPORT

which under Rule 45 of the European Patent Convention
shall be considered, for the purposes of subsequent
proceedings, as the European search report

Application Number

EP 04 07 7784

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 03/057207 A (SOUND PHARMACEUTICALS INCORPORATED; KIL, JONATHAN; LYNCH, ERIC, D) 17 July 2003 (2003-07-17)<br>* abstract; claims 5,7,23 *<br>* page 1, line 20 - page 2, line 18 *<br>* page 3, line 25 - page 9, line 2 *<br>----- | 1,2,5, 7-9 | A61K31/519<br>A61K31/52<br>A61K31/198<br>A61P27/16<br>A61P25/28 |
| X | WO 2004/000042 A (N.V. NUTRICIA; HAGEMAN, ROBERT, JOHAN, JOSEPH; VERLAAN, GEORGE) 31 December 2003 (2003-12-31)<br>* abstract; claims 3,17 *<br>* page 1, line 31 - page 2, line 13 *<br>* page 5, lines 4-11 *<br>* page 7, lines 15-17 *<br>* page 12, lines 5-17 *<br>-----<br><br>-/-- | 1-10 | |

TECHNICAL FIELDS
SEARCHED (Int.Cl.7)

A61K
A61P

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 31 March 2005 | A. Jakobs |

EPO FORM 1503 03.82 (P04C07)

**European Patent Office**  **PARTIAL EUROPEAN SEARCH REPORT**  Application Number

EP 04 07 7784

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | SPILLMANN M ET AL: "S-ADENOSYLMETHIONINE (ADEMETIONINE) IN PSYCHIATRIC DISORDERS HISTORICAL PERSPECTIVE AND CURRENT STATUS" CNS DRUGS, ADIS INTERNATIONAL, AUCKLAND, NZ, vol. 6, no. 6, 1996, pages 416-425, XP001027761 ISSN: 1172-7047 * abstract * * page 422, column 1, paragraph 7 - column 2, paragraph 1 * ----- | 1,3,5,7,9 | |
| X | EP 0 388 827 A (BIORESEARCH S.P.A) 26 September 1990 (1990-09-26) * the whole document * ----- | 1,3-10 | |
| X | WO 01/84961 A (N.V. NUTRICIA; KILIAAN, AMANDA, JOHANNE; HAGEMAN, ROBERT, JOHAN, JOSEP) 15 November 2001 (2001-11-15) * abstract; claims 8,17 * ----- | 1-5,7-9 | **TECHNICAL FIELDS SEARCHED** (Int.Cl.7) |
| X | WO 98/19690 A (BRISTOL-MYERS SQUIBB COMPANY) 14 May 1998 (1998-05-14) * page 3, line 18 - page 5, line 20; claims 1-22 * * page 6, line 14 - page 7, line 9 * ----- | 1,3-10 | |
| X | WO 00/25793 A (MERCK PATENT GMBH; BUCHHOLZ, HERWIG; MEDUSKI, JERZY, D) 11 May 2000 (2000-05-11) * abstract; claims 1-9 * * page 6, paragraph 2 * * page 8, paragraph 4 - page 9, paragraph 2 * ----- -/-- | 1,3-10 | |

EPO FORM 1503 03.82 (P04C10)

**European Patent Office**

## PARTIAL EUROPEAN SEARCH REPORT

Application Number

EP 04 07 7784

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | US 2003/148991 A1 (HAHNLEIN WOLFGANG P ET AL) 7 August 2003 (2003-08-07) * abstract * * paragraphs [0023], [0035], [0036], [0050], [5851] * ----- | 1,3-10 | |
| X | WO 02/32434 A (UNIVERSITA' DEGLI STUDI DI ROMA "LA SAPIENZA"; SCARPA, SIGFRIDO; FUSO,) 25 April 2002 (2002-04-25) * the whole document * ----- | 1,3-5, 7-9 | |
| X | US 6 420 342 B1 (HAGEMAN ROBERT JOHAN JOSEPH ET AL) 16 July 2002 (2002-07-16) * abstract; claims 1,12,18 * * column 5, lines 8-55 * * column 7, lines 59-65 * * column 12, lines 5-14 * ----- | 1,3-10 | |
| X | WO 02/071874 A (SOCIETE DES PRODUITS NESTLE S.A; MALNOE, ARMAND; PRIDMORE-MERTEN, SYLV) 19 September 2002 (2002-09-19) * abstract; claims 1,5,12 * ----- | 1-5,7-9 | |
| X | BOTTIGLIERI, TEODORO: "Ademetionine (S-adenosylmethionine) neuropharmacology: implications for drug therapies in psychiatric and neurological disorders" EXPERT OPINION ON INVESTIGATIONAL DRUGS , 6(4), 417-426 CODEN: EOIDER; ISSN: 0967-8298, 1997, XP008044814 * abstract * * page 422, column 1, paragraph 2 - column 2, paragraph 1 * ----- -/-- | 1,3-5, 7-9 | |

TECHNICAL FIELDS SEARCHED (Int.Cl.7)

**European Patent Office**

## PARTIAL EUROPEAN SEARCH REPORT

Application Number

EP 04 07 7784

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | LALONDE, ROBERT ET AL: "Effect of folic acid and folinic acid on cognitive and motor behaviors in 20-month-old rats" PHARMACOLOGY, BIOCHEMISTRY AND BEHAVIOR , 44(3), 703-7 CODEN: PBBHAU; ISSN: 0091-3057, 1993, XP008044825 * the whole document * ----- | 1,3-10 | |

TECHNICAL FIELDS SEARCHED (Int.Cl.7)

European Patent
Office

**INCOMPLETE SEARCH
SHEET C**

Application Number

EP 04 07 7784

Claim(s) searched incompletely:
        1-10

Claim(s) not searched:
            -

Reason for the limitation of the search:

Present claims relate to a compound defined by reference to the following
parameter:
a substance that increases 10-formyltetrahydrofolate, an ester thereof.
The use of these parameters in the present context is considered to lead
to a lack of clarity within the meaning of Article 84 EPC. It is
impossible to compare the parameters the applicant has chosen to employ
with what is set out in the prior art. The lack of clarity is such as to
render a meaningful complete search impossible. Consequently, the search
has been restricted to the individual compounds of claim 5 and their
salts.

EP 1 645 276 A1

**ANNEX TO THE EUROPEAN SEARCH REPORT
ON EUROPEAN PATENT APPLICATION NO.**                EP 04 07 7784

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2005

| Patent document cited in search report | | Publication date | Patent family member(s) | | Publication date |
|---|---|---|---|---|---|
| WO 03057207 | A | 17-07-2003 | AU | 2003202219 A1 | 24-07-2003 |
| | | | CA | 2472678 A1 | 17-07-2003 |
| | | | EP | 1471902 A1 | 03-11-2004 |
| | | | WO | 03057207 A1 | 17-07-2003 |
| | | | US | 2003162747 A1 | 28-08-2003 |
| | | | US | 2004220145 A1 | 04-11-2004 |
| WO 2004000042 | A | 31-12-2003 | AU | 2003247224 A1 | 06-01-2004 |
| | | | AU | 2003247225 A1 | 06-01-2004 |
| | | | EP | 1513419 A2 | 16-03-2005 |
| | | | WO | 2004000297 A1 | 31-12-2003 |
| | | | WO | 2004000042 A2 | 31-12-2003 |
| | | | US | 2004192615 A1 | 30-09-2004 |
| EP 0388827 | A | 26-09-1990 | IT | 1229203 B | 25-07-1991 |
| | | | AT | 97809 T | 15-12-1993 |
| | | | AU | 637086 B2 | 20-05-1993 |
| | | | AU | 5209290 A | 27-09-1990 |
| | | | CA | 2012876 A1 | 22-09-1990 |
| | | | DE | 69004837 D1 | 13-01-1994 |
| | | | DE | 69004837 T2 | 07-04-1994 |
| | | | DK | 388827 T3 | 21-03-1994 |
| | | | EP | 0388827 A1 | 26-09-1990 |
| | | | ES | 2062141 T3 | 16-12-1994 |
| | | | JP | 2062027 C | 10-06-1996 |
| | | | JP | 2289517 A | 29-11-1990 |
| | | | JP | 7091190 B | 04-10-1995 |
| | | | US | 5059595 A | 22-10-1991 |
| | | | ZA | 9002153 A | 28-12-1990 |
| WO 0184961 | A | 15-11-2001 | AU | 5511401 A | 20-11-2001 |
| | | | CA | 2408032 A1 | 15-11-2001 |
| | | | CN | 1440240 A | 03-09-2003 |
| | | | EP | 1282365 A2 | 12-02-2003 |
| | | | WO | 0184961 A2 | 15-11-2001 |
| | | | US | 2002040058 A1 | 04-04-2002 |
| WO 9819690 | A | 14-05-1998 | AU | 719290 B2 | 04-05-2000 |
| | | | AU | 5244298 A | 29-05-1998 |
| | | | CA | 2270780 A1 | 14-05-1998 |
| | | | EP | 0951293 A1 | 27-10-1999 |
| | | | JP | 2001504104 T | 27-03-2001 |
| | | | WO | 9819690 A1 | 14-05-1998 |
| | | | US | 6127370 A | 03-10-2000 |
| | | | US | 6008221 A | 28-12-1999 |

For more details about this annex : see Official Journal of the European Patent Office, No. 12/82

18

## EP 1 645 276 A1

## ANNEX TO THE EUROPEAN SEARCH REPORT
## ON EUROPEAN PATENT APPLICATION NO.

EP 04 07 7784

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2005

| Patent document cited in search report | | Publication date | Patent family member(s) | | Publication date |
|---|---|---|---|---|---|
| WO 0025793 | A | 11-05-2000 | AT | 235244 T | 15-04-2003 |
| | | | AU | 6202299 A | 22-05-2000 |
| | | | CA | 2348390 A1 | 11-05-2000 |
| | | | DE | 69906345 D1 | 30-04-2003 |
| | | | DE | 69906345 T2 | 12-02-2004 |
| | | | WO | 0025793 A1 | 11-05-2000 |
| | | | EP | 1124559 A1 | 22-08-2001 |
| | | | ES | 2194513 T3 | 16-11-2003 |
| | | | JP | 2002528507 T | 03-09-2002 |
| | | | US | 6514973 B1 | 04-02-2003 |
| US 2003148991 | A1 | 07-08-2003 | DE | 10022856 A1 | 15-11-2001 |
| | | | AU | 6742401 A | 20-11-2001 |
| | | | CA | 2408666 A1 | 08-11-2002 |
| | | | WO | 0185165 A2 | 15-11-2001 |
| | | | EP | 1289521 A2 | 12-03-2003 |
| | | | JP | 2003532674 T | 05-11-2003 |
| | | | ZA | 200209940 A | 09-12-2003 |
| WO 0232434 | A | 25-04-2002 | IT | RM20000556 A1 | 22-04-2002 |
| | | | AU | 1518702 A | 29-04-2002 |
| | | | CA | 2425514 A1 | 25-04-2002 |
| | | | EP | 1326620 A1 | 16-07-2003 |
| | | | WO | 0232434 A1 | 25-04-2002 |
| | | | US | 2004048827 A1 | 11-03-2004 |
| US 6420342 | B1 | 16-07-2002 | AU | 5685601 A | 20-11-2001 |
| | | | CN | 1429113 A | 09-07-2003 |
| | | | EP | 1282426 A1 | 12-02-2003 |
| | | | JP | 2003532679 T | 05-11-2003 |
| | | | WO | 0185178 A1 | 15-11-2001 |
| | | | US | 2002183263 A1 | 05-12-2002 |
| WO 02071874 | A | 19-09-2002 | BR | 0207948 A | 27-07-2004 |
| | | | CA | 2439078 A1 | 19-09-2002 |
| | | | WO | 02071874 A2 | 19-09-2002 |
| | | | EP | 1372412 A2 | 02-01-2004 |
| | | | JP | 2004519241 T | 02-07-2004 |
| | | | MX | PA03007802 A | 08-12-2003 |
| | | | NO | 20033941 A | 05-09-2003 |
| | | | PL | 363426 A1 | 15-11-2004 |
| | | | US | 2004047896 A1 | 11-03-2004 |

EPO FORM P0459

For more details about this annex : see Official Journal of the European Patent Office, No. 12/82